(19) Europäisches Patentamt / European Patent Office / Office européen des brevets

(11) **EP 3 290 074 A1**

(12) # EUROPEAN PATENT APPLICATION

(43) Date of publication:
**07.03.2018 Bulletin 2018/10**

(51) Int Cl.:
**A61M 5/168** (2006.01)  **G06F 19/00** (2018.01)
**A61M 39/10** (2006.01)  **A61M 5/142** (2006.01)

(21) Application number: **16186204.0**

(22) Date of filing: **30.08.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Nokia Technologies Oy**
**02610 Espoo (FI)**

(72) Inventor: **PETÄINEN, Katriina**
**36200 Kangasala (FI)**

(74) Representative: **Nokia EPO representatives**
**Nokia Technologies Oy**
**Karaportti 3**
**02610 Espoo (FI)**

(54) **INFUSION THERAPY**

(57)     A method comprising receiving a first indication indicating a disconnection between a connector and an infusion cannula, determining a duration of the disconnection, determining, based on the determined duration of the disconnection, an amount of infusion fluid not delivered because of the disconnection between the connector and the infusion cannula, and providing a second indication indicating the amount of infusion fluid not delivered.

Figure 6f

## Description

### Technical field

The present application generally relates to infusion therapy.

### Background

This section illustrates useful background information without admission of any technique described herein as representative of the state of the art.

Infusion therapy generally refers to administering medication through a cannula set to a human or animal body. For example, diabetes is often treated with insulin infusion therapy using infusion pumping to administer a desired amount of medicament. Insulin pump systems used by diabetics typically contain an insulin pump and an infusion set. An infusion set may include a cannula set for subcutaneous insertion and a tubing system to interface an insulin reservoir, a part of the infusion set for storing the insulin, where the tubing is connected to the insulin pump and serves to deliver insulin under the skin through the cannula set. Insulin pumps have greatly helped diabetics to avoid potentially severe consequences of insufficient insulin treatment by reducing the need to remember to inject new insulin doses, and also help people with needle phobia as they require penetration of the skin less frequently-for example only once in two or three days instead of multiple times per day if insulin is injected using a conventional syringe.

An infusion set is typically manufactured so that a user can detach and reattach the insulin pump and the cannula set. Thereby the insulin pump can be disconnected before bathing, for example, or for maintenance of the infusion pump. Detachment thus provides for protection of the pump and the avoidance of safety hazards. Some insulin pumps are equipped with a joining unit that can be used for attaching and detaching the pump and the cannula set. The joining unit is mounted in tubing between the insulin pump and the cannula set. The joining unit and/or the cannula set has a sealing order to avoid entry of air, water or dirt into the infusion set whilst the pump and the cannula set are disconnected from one another and thereby under the user's skin. On reconnection a fluid connection is reopened through the seal(s) between the connector and the cannula set.

### Summary

Various aspects of example embodiments are set out in the summary, the drawings, the detailed description and the claims.

According to various but not necessarily all embodiments of the invention there is provided a method for receiving a first indication indicating a disconnection between a connector and an infusion cannula, determining a duration of the disconnection, determining, based on the determined duration of the disconnection, an amount of infusion fluid not delivered because of the disconnection between the connector and the infusion cannula, and providing a second indication indicating the amount of infusion fluid not delivered.

According to various but not necessarily all embodiments of the invention, there is provided an apparatus comprising means for receiving a first indication indicating a disconnection between a connector and an infusion cannula, determining a duration of the disconnection, determining, based on the determined duration of the disconnection, an amount of infusion fluid not delivered because of the disconnection between the connector and the infusion cannula, and providing a second indication indicating the amount of infusion fluid not delivered.

The apparatus may be for example a medical device such as an infusion pump system or an infusion pump.

According to various but not necessarily all embodiments of the invention, there is provided an apparatus comprising a memory and software, configured to, with a processor circuitry, cause the apparatus to perform at least the following: receiving a first indication indicating a disconnection between a connector and an infusion cannula, determining a duration of the disconnection, determining, based on the determined duration of the disconnection, an amount of infusion fluid not delivered because of the disconnection between the connector and the infusion cannula, and providing a second indication indicating the amount of infusion fluid not delivered.

The apparatus may be for example a medical device such as an infusion pump system or an infusion pump.

According to various but not necessarily all embodiments of the invention, there is provided a computer program product comprising computer program code stored in a non-transitory memory medium, the computer program code being configured to cause an apparatus, when executing the program code by a processor circuitry, to perform at least the following: receiving a first indication indicating a disconnection between a connector and an infusion cannula, determining a duration of the disconnection, determining, based on the determined duration of the disconnection, an amount of infusion fluid not delivered because of the disconnection between the connector and the infusion cannula, and providing a second indication indicating the amount of infusion fluid not delivered.

## Brief Description of the Figures

For a better understanding of various examples that are useful for understanding the brief description, reference will now be made by way of example only to the accompanying drawings in which:

Figure 1 illustrates an example embodiment of an infusion pump system;
Figure 2 illustrates an example embodiment of an infusion pump;
Figure 3 illustrates an example embodiment of a tubing, a cannula set and a joining unit;
Figure 4 illustrates an example embodiment of a joining unit;
Figure 5 illustrates an example embodiment of a connection detector;
Figure 6a illustrates a situation in which a connector is disconnected from an infusion cannula;
Figure 6b illustrates an example embodiment of an indication indicating a detected disconnection;
Figures 6c- 6e illustrate examples of periods of disconnection;
Figure 6f illustrates an example of a user interface;
Figure 7 is a flow diagram illustrating an example of a method.

## Detailed Description

If multiple daily doses of medicament are needed, an infusion pump system may be used. An apparatus such as an infusion pump system is a medical device that may be used for the administration of an infusion fluid such as insulin, antibiotics or other suitable medicament. The configuration of an infusion pump system may vary from one system to another. An example embodiment of an infusion pump system comprises an infusion pump, which comprises one or more control unit, and a power source, for example a battery. Example embodiments of the infusion pump system may further comprise an infusion fluid reservoir for the infusion fluid and a cannula set for subcutaneous insertion under skin and a tubing to interface the insulin reservoir to the cannula set. It is possible that some parts of the infusion pump system are disposable and replaceable, for example the infusion fluid reservoir. The tubing may be long enough to enable a user to carry the infusion pump attached to his/her waist or in a pocket.

Other configurations are also possible. For example, in some embodiments, an infusion pump may be attached to a cannula set without a tubing system, for example if the infusion pump or the infusion fluid reservoir itself comprises a connector and is attached with the connector directly to the cannula set. In this configuration, the infusion pump would normally be located next to the user's skin. One of the benefits achieved by using an infusion pump may be that multiple, individual, daily injections of the infusion fluid may be avoided and the delivery of the infusion fluid may be more accurate and reliable. If the infusion pump system further includes capabilities of measuring medical conditions, such as blood glucose monitoring for example, the amount of the infusion fluid, such as insulin for example, to be delivered may be adjusted based on the measurement results -for example in real time.

An infusion pump may be attached to a user using a cannula set and a joining unit connected to the cannula set that enables joining the cannula set to a tubing that connects an infusion pump and the cannula set. The user may want to release the infusion pump every now and then for a while for example if the user wishes to exercise or go swimming. In such a situation, the cannula set can remain on the user whilst some or all of the rest of the infusion pump system (e.g. the joining unit, tubing and infusion pump) is removed. While the infusion pump is disconnected from the cannula set, infusion fluid cannot be delivered. It is also possible that the joining unit is disconnected from the cannula set by accident and thereby the connection between the cannula set and the joining unit is lost accidentally. It may be useful for a user to know for how long the joining unit was disconnected and/or if some amount of the infusion fluid has not been delivered because of the disconnection.

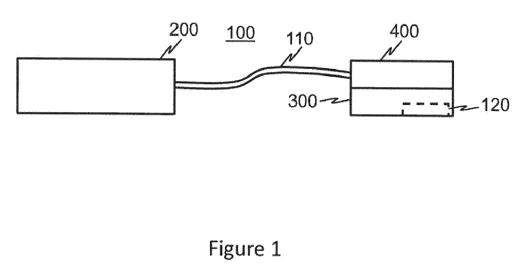

Figure 1 shows an example embodiment of an infusion pump system 100. The infusion pump system 100 comprises an infusion pump 200, a cannula set 300 and a joining unit 400 for connecting the infusion pump 200 and the cannula set 300 and a tubing 110 for interconnecting the joining unit 400 and the infusion pump 200. The infusion pump system 100 may further comprise a monitoring system 120 for monitoring the status of the user wearing the cannula set 300, for example a continuous glucose monitor. For example, the infusion pump system 100 may be an insulin delivery system, wherein the infusion pump system 100 may further comprise a monitoring system 120 for continuously monitoring the glucose level of the user integrated to the cannula set 300. A sensor of the monitoring system 120 for continuous monitoring of the glucose level of the user may be a separate unit, i.e. not integrated to cannula set 300 or alternatively, it may be an integrated part of the cannula set 300.

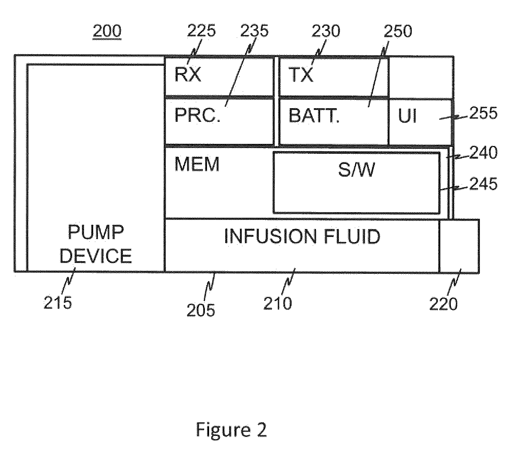

Figure 2 shows a block diagram of the infusion pump 200 of an example embodiment. The infusion pump 200 may comprise an infusion fluid reservoir 205 for storing infusion fluid 210, which may be a medicament for example, and an output port 220 for connecting with the tubing 110. The infusion pump device 215, which comprises the part of the infusion pump that is the physical pumping mechanism, may be configured to deliver a particular dose (e.g. a bolus or continuous infusion having a predefined volume or mass or, in the case of an infusion, a predefined rate of administration) of the infusion fluid 210 to the tubing 110 via the output port for delivery to the cannula set 300 through the joining unit 400. The infusion pump 200 may also comprise a wireless receiver 225, a wireless transmitter 230, a processor circuitry 235 configured to control operation of the infusion pump 200, a memory 240 comprising computer program code 245 that may be executed by the processor circuitry 235, and a battery 250 for powering the operation of the infusion pump 200.

In some example embodiments, the infusion fluid reservoir 205 forms a part of the infusion pump 200. For example, the infusion fluid reservoir 205 may be an ampoule comprising a piston that is actuated by an actuator for dosing the infusion fluid. In another example embodiment, the infusion storage 205 may be a flexible unit such as a bag that is compressed by a desired volume with an actuator such as a piston. Figure 2 presents the block diagram schematically and for ease of illustration and therefore the fluid reservoir 205 is drawn as a rigid tank, whereas in other example embodiments the fluid reservoir 205 may take any appropriate form, for example it may be rigid or deformable, it may be a disposable unit, and/or it may be an ampoule. The fluid reservoir 205 may be made, for example, from plastics, glass or any other suitable material.

An infusion pump 200 may further comprise a user interface 255 to enable receiving input from the user and/or to enable providing information to the user. The user interface 255 may comprise one or more of a display, loudspeaker, haptic feedback unit, speech synthesizer, touch screen, button, keypad, microphone, and speech recognizing circuitry. In some embodiments a user interface for use with an infusion pump may comprise components that are physically separate from the infusion pump 200 and are connected to the infusion pump 200 by means of a wireless or wired connection. It is also to be noted that in some example embodiments the infusion pump 200 may in general be connected to an external device by means of a wireless or wired connection. An external device is another device that is an entity on its own and can function on its own and may be connected to other devices. The external device could be for example a mobile phone, a tablet computer, a smart watch, a wearable device, a computer or a head-mounted display.

In some example embodiments, the processor circuitry235 may comprise, for example, any one or more of: a Master Control Unit (MCU); a microprocessor; a Digital Signal Processor (DSP); an Application Specific Integrated Circuit (ASIC); a Field Programmable Gate Array (FPGA); and a microcontroller.

In some example embodiments, the infusion pump 200 may be configured to deliver insulin. In other example embodiments, the infusion pump 200 may be configured to deliver pain medication. In yet other example embodiments, the infusion pump unit 200 may be an antibiotic delivery system configured to deliver antibiotics. In other example embodiments the infusion pump unit 200 may be configured to deliver another medicament, or a combination of medicaments.

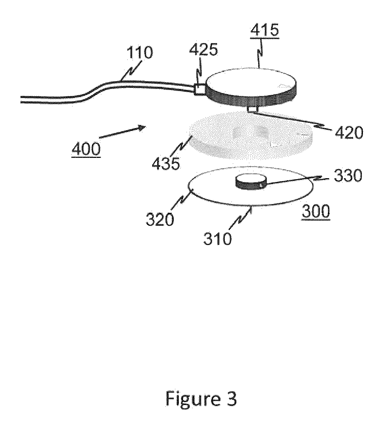

Figure 3 illustrates an example embodiment of the tubing 110, the cannula set 300 and the joining unit 400. The joining unit 400 may comprise a connector 415 and an intermediate part that appears in Figure 3 as a separate housing 435. The separate housing 435 defines a hole through which the connector 415 can be locked to the cannula set with a mechanical connection either to the infusion cannula 310 or by coupling to other part(s) of the cannula set 300 that are mechanically coupled with the infusion cannula 310. The connector 415 can further be connected to the infusion cannula 310 with a fluid connection, which enables flow of an infusion fluid 210.

In the example embodiment illustrated in Figure 3, the cannula set 300 comprises an infusion cannula 310 that is configured to deliver the infusion fluid 210 into subcutaneous tissue of a user of the cannula set 300. The cannula set 300 further comprises a skin attachment member 320 configured to maintain the cannula set 300 in place for example for a plurality of hours. In this example embodiment, the skin attachment member 320 comprises adhesive material for attaching the member 320 to the skin. The cannula set 300 may comprise a connector port 330 configured to form a fluid connection with the infusion pump device 215 through the connector 415, when the connector 415 is attached to the infusion cannula 310.

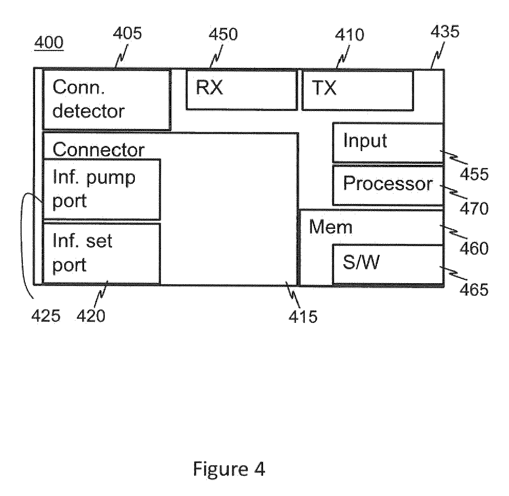

Figure 4 shows a block diagram of a joining unit 400 of an example embodiment. The joining unit 400 comprises a connection detector 405 configured to detect whether an infusion pump 200 is connected to an infusion cannula 310 with the connector 415; and a transmitter 410 (for example a wireless transmitter) configured to transmit to an infusion pump unit 200 an indication in response to detection by the connection detector 405 whether or not the connector 415 is connected to the infusion cannula 310 to form a fluid connection.

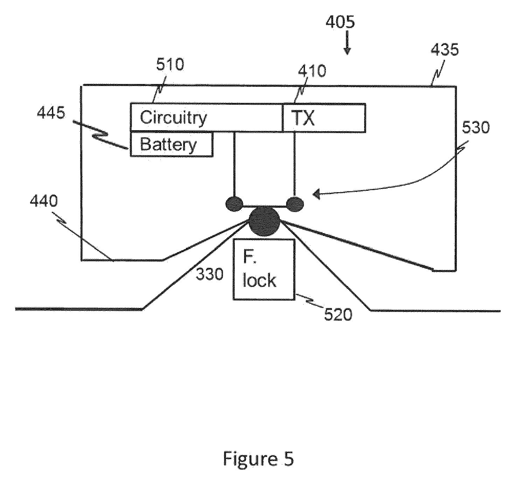

The connector 415 is connectable to the output port 220 via, for example, a flexible tubing. In an example embodiment, the connector port 330 comprises a form lock 520, as illustrated in Figure 5, configured to user-releasably connect the connector 415 to the infusion cannula 310. The form lock 520 may be configured to form a match between the infusion cannula 310 and the connector 415. When the form lock 520 is in a correct position or orientation, the infusion cannula 310 and the connector 415 are correctly aligned and in the fluid connection. Then, the infusion fluid 210 may be pumped by the infusion pump 200 through the infusion cannula 310.

In some example embodiments, the housing 435 is attachable to the connector 415 to form a combination that can then be connected to the cannula set 300. Alternatively or additionally, the housing 435 may be attachable to the cannula set 300 (for example using a further form lock) before the connection between the connector 415 and the infusion cannula 310 takes place.

In some example embodiments, there is no form lock directly between the cannula set 300 and the connector 415, but instead these two are attached to each other by attaching the housing 435 separately to each of the cannula set 300 and the connector 415, using two different form locks, for example.

In some example embodiments, the joining unit 400 may be configured such that the connector 415 is configured to enable the connection detector 405 to detect whether the connector 415 is connected to the infusion cannula 310. The connector 415 may also be configured to inhibit the connection detector 405 from detecting that the connector 415 is connected to the infusion cannula 310 unless the connector 415 forms a fluid connection with the infusion cannula 310.

In some example embodiments, the wireless transmitter 410 comprises a radio transmitter. The wireless transmitter 410 can be for example a low power wireless connection means, such as Bluetooth Low Energy (BLE) or Near Field Communications (NFC), and as a battery-powered or passive radio-powered device. Alternatively, or additionally, the wireless transmitter 410 may comprise an ultrasound transmitter, or any suitable radio frequency transmitter. The wireless transmitter can be used, for example, when connecting to a physically separate component, which, in some example embodiments, may form part of the user interface 255.

In some example embodiments, the connection detector 405 may be water-proof, water-resistant or hermetically sealed. The connection detector 405 comprises, in some example embodiments, the wireless transmitter 410. In some example embodiments, the housing 435 comprises a wall 440 separating the connection detector 405 from the infusion cannula 310. The connection detector 405 may be configured to detect the connection through the wall 440. In some example embodiments, the wall 440 is flexible. In some example embodiments, the connection detector 405 is configured to detect the connection based on a deformation of the wall 440. In some example embodiments, the form lock 520 is configured to deform the wall 440 whilst the connector 415 is connected to or disconnected from the infusion cannula 310, or to deform the wall during transitions between connection and disconnection.

In some example embodiments, the housing 435 may need to be oriented in a particular angular orientation with regard to the connector 415 and/or the cannula set 300 in order for the connection detector 405 to function properly. Therefore, an orientation guide may be provided, such as physical shapes that form counterparts to each other and thereby necessitate proper orientation between the housing 435 and an adjacent element selected from the connector 415 and the cannula set 300. Alternatively, the connection detection may be implemented such that the housing 435 can be attached in any angular orientation with regard to the connector 415 and/or the cannula set 300 (for example, through detection of flexing of a flexible wall 440).

In the example embodiment illustrated in Figure 5, the housing 435 contains a battery 445 for powering at least the wireless transmitter 410 and circuitry 510 for controlling the operation of the connection detector 405. In some example embodiments, the connection detector 405 is configured to power the wireless transmitter 410 using the battery 445 when the connection detector 405 detects that the connector 415 is connected to the cannula 310.

The connection detector may comprise a switch 530, as illustrated in Figure 5, or other sensor circuitry that is configured to detect the connection through the wall 440. In an example embodiment, the switch is formed of conductive surface 540 of the housing 435. When the form lock 520 presses the flexible wall 440 at a certain position, the surface 540 forms an electrical connection indicating that the connector 415 is properly connected to the infusion cannula 310 to form a fluid connection, as illustrated in Figure 5. In some other example embodiments there may be provided (instead or in addition to the switch) a magnetic sensor, an inductive sensor, a capacitive sensor, an optical sensor, or any combination thereof.

The battery 445 may be disposable or alternatively, it may be rechargeable. The battery 445 may be integrated into or housed in the joining unit 400. In some example embodiments, the connection detector 405 is integrated into the connector 415. If the connection detector 405 is integrated to the connector 415, and the form lock 520 is integrated to the connection port 330 of the cannula set 300, the separate housing 435 may not be needed. The connection detector 405 may be integrated into the housing 435.

In some example embodiments, the wireless transmitter 410 may be configured to transmit an indication of whether a connection is present in response to a detection by the connection detector 405 that the infusion pump device 215 is connected to the infusion cannula 310. In some example embodiments, the transmitter 410 transmits the indication periodically, an effect of which may be to reduce power consumption and e.g. save power of the battery 445. The transmitter 410 may be configured to transmit the indication before the infusion pump 200 delivers a dose of the infusion fluid 210 through the infusion cannula 310.

In some example embodiments, the joining unit 400 further comprises a receiver 450 configured to receive, from the infusion pump 200, an interval configuration for delivering doses of the infusion fluid 210 that specifies (or otherwise enables the joining unit 400 to calculate) when each dose will be delivered. The configuration may then be used to configure the transmitter 410 to send, according to the interval configuration, indications indicating if there is a connection. For example, the receiver 450 may be a wireless receiver and the joining unit 400 can be configured to transmit the indications at a desired rate. For example, the infusion pump 200 may be configured to deliver a set of doses at a set rate such as once per ten seconds or by some multiple of the set rate, such as twice or thrice the rate. Before a delivery of a dose, the connection detector 405 may verify the fluid connection to the infusion pump 200 exists by transmitting an indication indicating if a connection is detected or not. In some example embodiments, the joining unit 400 is configured to adjust the periodic transmitting of the indications indicating a connection according to a pumping interval of the infusion pump 200. In some example embodiments, the infusion pump 200 may be configured to verify, from the received indication from the joining unit 400, that a fluid connection exists between the connector 415 of the joining unit 400 and the infusion cannula 310 of the cannula set 300 before a dose of the infusion fluid 210 is delivered. It may also be possible that the infusion pump 200 is initiated to deliver a dose of the infusion fluid 210 that is not according to a schedule or a set interval but in accordance to an input received from a user or in response to a sensor measurement for example. In such a case the infusion pump 200 may be configured to prompt the joining unit 400 to transmit an indication before delivering the dose of the infusion fluid 210.

In some example embodiments, the joining unit 400 may comprise an input 455 for receiving feedback from a sensor 120, for example a blood glucose sensor. The sensor may be for example a Continuous Glucose Monitoring (CGM) sensor. The input 455 may comprise a wired or wireless input means such as, for example, a wireless receiver 450 or a wired analog or digital input. In an example embodiment, the transmitter 410 is configured to transmit the feedback (such as a sensed glucose level indication) to the infusion pump 200 for adjustment of one or more doses of the infusion fluid 210 to be delivered by the infusion pump 200. The sensor 120 may be comprised by the cannula set 300.

In some example embodiments, the joining unit 400 further comprises a memory 460 storing computer program code 465. The joining unit 400 may further comprise a processor circuitry 470 for controlling the operation of the joining unit 400 by execution of the computer program code 465. The processor circuitry 470 may comprise, for example, any one or more of: a Master Control Unit (MCU); a microprocessor; a Digital Signal Processor (DSP); an Application Specific Integrated Circuit (ASIC); a Field Programmable Gate Array (FPGA); and a microcontroller.

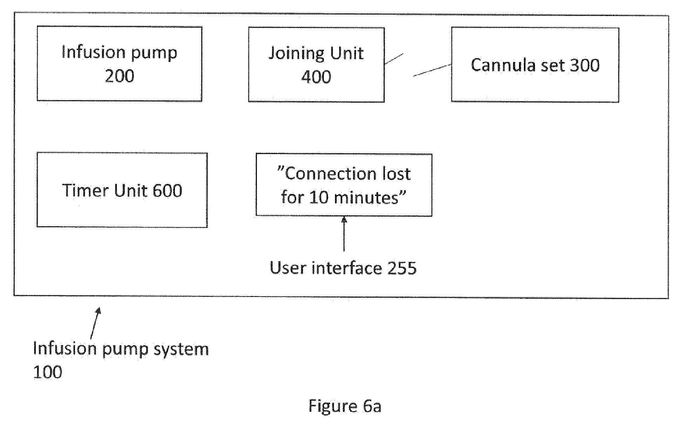

Figure 6a illustrates an example of a situation in which the connector 405 has been disconnected from the infusion cannula 310 and an indication has been generated to indicate this. In this example embodiment, the connector 405 and the cannula set 300 are parts of an infusion pump system such as the infusion pump system 100. This is for illustration purpose only and it is to be noted that other suitable infusion pump systems that enable detection of the disconnection between a connector and an infusion cannula may be used.

In this example embodiment, the infusion pump system 100 comprises means for detecting a duration of time. This capability may be achieved by utilizing a physical timer unit 600 such as a real time clock module, or alternatively a software timer unit 600 implemented in computer program instructions which, when executed by a processor circuitry, cause the duration of time to be measured. In some embodiments the timer unit 600 may determine a duration of time based on signals received from a clock external to the infusion pump system 200, for example based on radio signals received from an external device. When a disconnection between the connector 405 and the infusion cannula 310 has occurred, the duration of this disconnection may be determined by the timer unit 600. In some example embodiments, the current duration of the disconnection is output to a user interface 255 in real-time. Additionally or alternatively, a total duration of the disconnection is output to the user interface 255 only after the end of the disconnection. Other information regarding the delivery of the infusion fluid 210 may be output to the user interface 255, for example in addition to the duration of a disconnection.

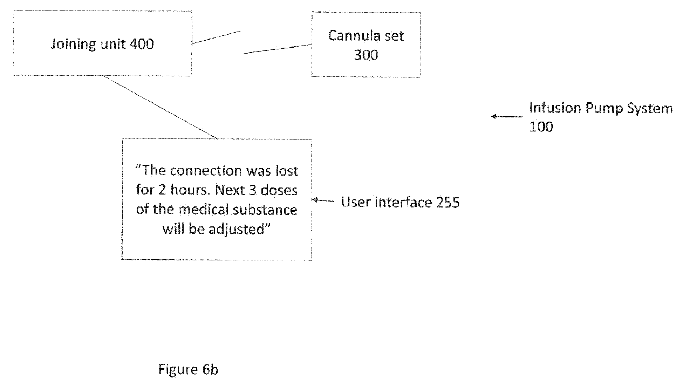

Figure 6b illustrates an example embodiment in which an indication indicating the detected disconnection is displayed to the user. A display displaying the indication may be part of the infusion pump system 100 and comprised by the user interface 255. Alternatively or additionally, the indication indicating the detected disconnection may be transmitted to be displayed on an external display that does not form part of the infusion pump system 100. Examples of such external displays may comprise for example, a display of a mobile device, a display of a wearable device such as a near-eye display or wrist worn device, a display included in a vehicle and/or household appliance etc. A wireless transmitter 410 of the infusion pump system 100 may be utilized if the indication indicating the disconnection is to be displayed on an external display. The wireless transmitter 410 may transmit data comprising indications of disconnections either directly to an external display or a device comprising such a display, or to a cloud service or other intermediary, which then provides the indication to the external device for display.

In some example embodiments, the infusion pump system 100 may be configured to deliver different types of doses of the infusion fluid 210. For example, the infusion pump system 100 may be configured to continuously deliver the infusion fluid 210 at a constant basal rate. A basal rate may be configured by a user or it may be pre-configured, for example by a supplier of the infusion pump system 100, a medicament for use by the infusion pump system 100, or by a medical practitioner treating the user. The infusion pump system 100 may additionally be configured to deliver discrete amounts of the infusion fluid 210 as bolus doses. For example, if the infusion pump system 100 is used to deliver insulin as the infusion fluid 210, a bolus dose may be delivered proximate to a meal time to control the rise in blood glucose of a user. A bolus dose may be delivered as a single administration of medicament over a short period of time or as an extended bolus. The extended bolus, e.g. dual wave bolus, involves delivering percentage of the total bolus as an initial administration over a very short time period, with the remainder of the bolus dose delivered slowly over a period of time.

In some example embodiments, the indication indicating a detected disconnection may comprise (but is not limited to), an indication of the duration of the disconnection and/or the amount of infusion fluid 210 undelivered because of the disconnection. The indication may further comprise a recommendation for an action to be taken by the user, for example administering a bolus dose and/or reconnecting the cannula. The indication may be in the form of a sound, vibration, displayed text or any other suitable form of notification to a user. The indication may be provided once, continuously, or periodically, and may be provided until the disconnection is over and/or until the indication is acknowledged by a user. The amount of infusion fluid 210 undelivered during the disconnection may be determined by the insulin pump system 100. Additionally or alternatively, any other suitable means may be used for the determining purposes, such as a smartphone, cloud computing means, smart watch etc. In some example embodiments, the amount of infusion fluid 210 undelivered during the disconnection may comprise at least one of a bolus, basal and/or extended bolus delivery dose. For example, if the infusion pump 200 is configured to deliver basal doses at a constant basal rate (the amount of the infusion fluid 210 to be delivered per time unit), the amount of infusion fluid 210 undelivered during a disconnection (i.e. the underdose) may be determined by multiplying this basal rate by the duration of the disconnection. In this example embodiment, the basal rate is constant during the disconnection, but adapted calculations may be used to account for variation in the rate at which an infusion is delivered, or to account for bolus doses.

$$Amount\ of\ underdose = basal\ rate * duration\ of\ disconnect.$$

For example, when the infusion pump 200 has been configured to vary the basal rate during the disconnection period t1-t2, the total amount of underdose (Atot) of the infusion fluid 210 during a disconnection may therefore be determined to be:

$$Atot = \int_{t1}^{t2} B(t)\ dt$$

Where $B$ is the basal rate as a function of time $t$.

In a further example embodiment, the determination of the underdose of the infusion fluid 210 may be performed based on data referring to different basal rates during different time periods. For example, Table 1 below lists timed changes to a basal rate B with which an infusion pump 200 has been configured. At each time in the table the infusion pump 200 is configured to change to the associated basal rate.

Table 1. Insulin basal rate B delivery setups

| Basal rate (International Units of Insulin IU/h) | Time |
|---|---|
| 0.5 | 00:00 |
| 1 | 08:30 |
| 3 | 15:00 |
| 1 | 18:45 |

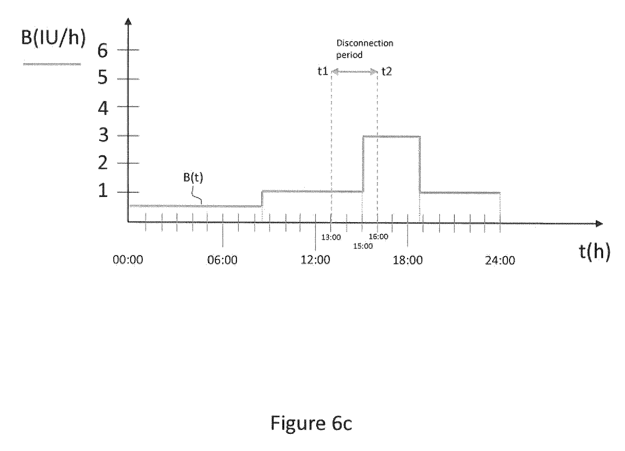

In this example, a connector 415 of a joining unit 400 of the infusion pump 200 has been disconnected from a cannula set 300 at 13:00 and connected back at 16:00 as depicted in Figure 6c. It is to be noted that the disconnection and reconnection may happen at any time, i.e. not restricted to the clock hour of this example embodiment. The clock hour in this example embodiment has been selected here for the clarity for this example only. The total amount of underdose (*Atot)* of infusion fluid 210 in this example embodiment may be determined using the following equation:

$$Atot = \begin{cases} \int_{t1}^{t2} B(t) \; dt \\ B(t) = \begin{cases} 0,5 \; IUh^{-1}, 00{:}00 \leq t < 08{:}30 \\ 1 \; IUh^{-1}, 08{:}30 \leq t < 15{:}00 \\ 3 \; IUh^{-1}, 15{:}00 \leq t < 18{:}45 \\ 1 \; IUh^{-1}, 18{:}45 \leq t < 24{:}00 \end{cases} \end{cases}$$

$$= \int_{13:00}^{15:00} B(t) \, dt + \int_{15:00}^{16:00} B(t) \, dt$$

$$= 1 \; IUh^{-1} \times 2 \; h + 3 \; IUh^{-1} \times 1 \; h = 5 \; IU$$

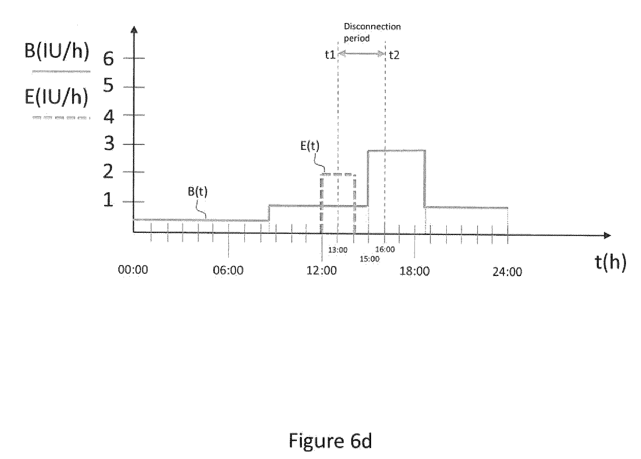

In a further example embodiment, depicted in Figure 6d, the infusion pump 200 is configured to deliver an extended bolus $E$ in addition to basal dose $B$. A disconnection then occurs and the infusion fluid 210 cannot be delivered according to the configuration. The configuration for the extended bolus is 2 IUh$^{-1}$ at 12:00-14:00. The total amount of underdose ($Atot$) during the disconnection period at 13:00 -16:00 (t1 to t2) can now be determined as

$$Atot = \int_{t1}^{t2} B(t) \; dt + \int_{t1}^{t2} E(t) \, dt$$

$$Atot = \int_{13:00}^{15:00} B(t) \, dt + \int_{15:00}^{16:00} B(t) \, dt + \int_{13:00}^{14:00} E(t) \, dt + \int_{14:00}^{16:00} E(t) \, dt$$

$$= 1 \; IUh^{-1} \times 2 \; h + 3 \; IUh^{-1} \times 1 \; h + 2 \; IUh^{-1} \times 1 \; h + 0 \; IUh^{-1} \times 2 \; h = 7 \; IU$$

Where the $B(t)$ is the basal as a function of time and $E(t)$ is the extended bolus as a function of time.

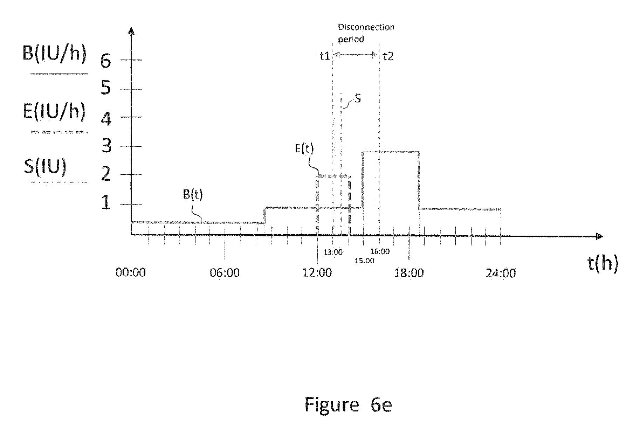

In an example embodiment, depicted in Figure 6e, the infusion pump 200 has been configured to deliver an extended bolus $E$, a one shot bolus $S$ and a basal $B$ while the disconnection occurs. Bolus $S$ is 5 IU in this example. The amount of underdose ($Atot$) in this example embodiment may be determined as

$$Atot = \int_{t1}^{t2} B(t) \; dt + \int_{t1}^{t2} E(t) \, dt + S$$

$$= 1 \; IUh^{-1} \times 2 \; h + 3 \; IUh^{-1} \times 1 \; h + 2 \; IUh^{-1} \times 1 \; h + 0 \; IUh^{-1} \times 2 \; h + 5 \; IU$$

$$= 12 \; IU$$

The content of an indication indicating a disconnection may be user-defined or predetermined by a manufacturer or a doctor for example, or a combination of both. For example, a manufacturer may define the indication to include information regarding the duration of the disconnection and whether any amount of the infusion fluid 210 was not delivered due to the disconnection and the user may define the format and some additional data such as date and time for example, or any other suitable combination may be possible. Alternatively, the user may only wish to see if any amount of the infusion fluid 210 was not delivered and therefore defines the indication to include only this information. Other indication definitions may also be used, and the choice of indication content will likely vary according the application of the infusion pump system 100.

In some example embodiments the duration of disconnect and/or the amount of infusion fluid 210 undelivered as a result of the disconnection, (i.e. the underdose), may be indicated in real-time during the disconnection. In some example embodiments, an infusion pump 200 may keep a record of the historical values of the duration of disconnections and/or amount of infusion fluid 210 not delivered during the disconnection periods. An infusion pump 200 may keep a record of the historical values of the delivered infusion fluid 210. Retained historical values of either or both the undelivered and delivered infusion fluid, and/or of the duration of connections and/or disconnections may be comprised in an indication. For example, an indication may inform a user of the total duration of disconnections during a 24 hour period, and the total amount of infusion fluid that remained undelivered as a result of those disconnections.

The above examples of dose types, frequency, duration, timing, etc. is provided purely by way of illustrative example. It will be understood that real-world use of an infusion pump system will follow dosing regimens that are appropriate for the user and their medical condition.

In some example embodiments, the infusion pump system 100 may deliver an amount of infusion fluid 210 to allow for the amount not delivered during a disconnection by using an adjusted amount of the infusion fluid 210 after reconnection. This adjusted amount of the infusion fluid may be delivered as one or more bolus doses (either additional bolus doses or by increasing the amount of already scheduled bolus doses) or by adjusting the basal rate to higher level for a certain time period, for example. Any suitable combination of increased and/or additional bolus doses and an increased basal rate may be used.

The additional amount of infusion fluid 210 that is to be delivered and the amount not delivered during the disconnection will not necessarily be equal. For example, for some applications calculation of the additional dosing of infusion fluid may be a simple question of selecting an appropriate dosing regimen (e.g. increased basal rate and/or one or more bolus doses) that will provide exactly the amount of infusion fluid 210 that was undelivered during disconnection(s). However, it may be that factors such as the duration of the disconnection(s), the total amount of infusion fluid 210 undelivered, the particular needs of the user, and/or the nature of the infusion fluid 210 (e.g. the type of medicament and guidelines for its administration) require a different additional amount of infusion fluid 210 to be administered. The calculation of the optimal additional amount will vary according to the use case and may be based on the amount of undelivered infusion fluid 210 and/or other factors. One factor that may influence the additional amount is a physiological measurement of the user (e.g. blood glucose level). The adjusted amount may even be user-configured rather than determined purely automatically by the infusion pump 200 - for example, the user may be provided with an option to confirm that an automatically calculated adjusted amount of the infusion fluid 210 should be delivered, or may manually input an additional amount that he or a medical practitioner wish to be administered.

The infusion pump 200 may be configured to automatically determine a suitable adjusted amount of the infusion fluid 210, to determine how it is to be delivered and to automatically deliver the adjusted amount of the infusion fluid once the disconnection has ended. In some example embodiments, a part of the adjusted amount of the infusion fluid 210 may be delivered immediately, and rest may be delivered by adjusting the basal rate. The adjusted amount of the infusion fluid 210 may also be delivered by so called extended delivery, where the adjusted amount of the infusion fluid 210 is distributed to be periodically delivered within a certain time period.

In some instances, the disconnection may have taken place accidentally and the user has failed to notice an indication indicating that the disconnection has happened. Once the user realizes that the connector 415 is not connected to the infusion cannula 310, the user may look at a display, which is comprised in the user interface 255 of the infusion pump system 100. The display may indicate to the user the duration of the disconnection, the amount of infusion fluid 210 not delivered and a suggestion that the adjusted amount of the infusion fluid 210 is not delivered at once, but instead the basal rate is adjusted higher such that the missing amount is evenly delivered and after the adjusted amount of the infusion fluid 210 has been delivered, to adjust the basal rate back to the default value.

In an example use case, the user may be exercising. To prevent damage to the infusion pump system 100 or trauma or inconvenience to the user during the exercise, the joining unit 400 is disconnected from the cannula set 300 by the user. As part of this the connector 415 is disconnected from the infusion cannula 310 by the user and an indication indicating the detected disconnection is transmitted to the user's smart watch. This indication indicates to the user that the disconnection has occurred. As the user is exercising, he decides not to immediately restore the connection but instead to wait until a more convenient time. Once the user restores the connection, an indication indicating the duration of the disconnection and if any amount of the infusion fluid 210 was not delivered because of the disconnection is transmitted to the smart watch of the user. Alternatively or additionally, the infusion pump system 100 may cause the smart watch to tell the user when the next dose of the infusion fluid 210 is to be delivered such that the user know by when the connection is to be restored in order to avoid any amount of the infusion fluid 210 not to be delivered.

In some example embodiments, configurations of the infusion pump 200 may have been stored to an external device. The external device may be a smart watch, a sports wearable (e.g. a fitness tracker such as a motion sensing wristband), a mobile phone or any other suitable device. Indications indicating at least the disconnection, duration of disconnection and/or infusion fluid 210 underdose may be transmitted to the external device. This may be beneficial in such situations where it is inconvenient for indications to be provided to the user via the insulin pump 200, for example because the user has disconnected the insulin pump 200 and is not carrying it with him, because the insulin pump 200 lacks functionality to enable it to provide the indication to the user, or because delivery via the external device is more convenient and/or discreet for the user. For example, providing the indication to the user via an external device such as a smart watch or mobile phone may enable the user to receive the notification without revealing to people in his vicinity that he uses an infusion pump system.

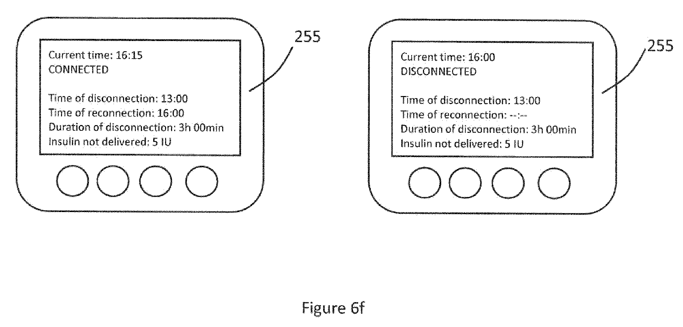

Figure 6f illustrates an example embodiment in which the user interface 255 is configured to display an indication indicating if there is a disconnection or not and also if there has been a disconnection. The indication further indicates the duration of the disconnection as well as an amount of insulin not delivered which the user interface 255 is further configured to display.

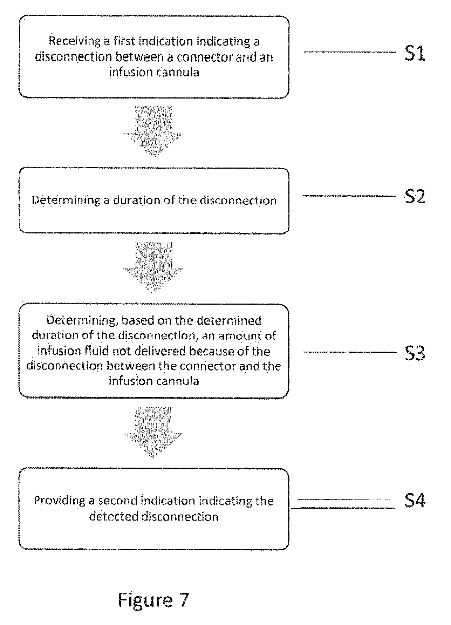

Figure 7 illustrates a flow chart according to an example embodiment. S1 comprises receiving a first indication indicating a disconnection between a connector and an infusion cannula. S2 comprises determining a duration of the disconnection. S3 comprises Determining, based on the determined duration of the disconnection, an amount of infusion fluid not delivered because of the disconnection between the connector and the infusion cannula. S4 comprises determining, based on the determined duration of the disconnection, an amount of infusion fluid not delivered because of the disconnection between the connector and the infusion cannula.

The effects that the example embodiments described above comprise at least providing a robust technique for monitoring connection status of a user releasable connection between an infusion pump and a cannula set.

Although various aspects of the invention are set out in the independent claims, other aspects of the invention comprise other combinations of features from the described example embodiments and/or the dependent claims with the features of the independent claims, and not solely the combinations explicitly set out in the claims.

It is also noted herein that while the foregoing describes example embodiments of the invention, these descriptions should not be viewed in a limiting sense. Rather, there are several variations and modifications which may be made without departing from the scope of the present invention as defined in the appended claims.

## Claims

1. A method comprising:

   receiving a first indication indicating a disconnection between a connector and an infusion cannula,
   determining a duration of the disconnection,
   determining, based on the determined duration of the disconnection, an amount of infusion fluid not delivered because of the disconnection between the connector and the infusion cannula, and
   providing a second indication indicating the amount of infusion fluid not delivered.

2. A method according to claim 1, wherein the disconnection between the connector and the infusion cannula is a disconnection of a fluid connection.

3. A method according to claim 1 or 2, wherein the second indication comprises at least one of a visual, audio or haptic form of indicating.

4. A method according to claim 3, wherein the second indication is provided by an infusion pump system that comprises the connector, the infusion cannula and an infusion pump.

5. A method according to any of claims 2 to 4, wherein providing the second indication comprises sending the indication to an external device.

6. A method according to claim 5, wherein the sending is over a wireless connection.

7. A method according to any previous claim, wherein the second indication comprises user-defined content.

8. A method according to any previous claim wherein the connector is part of a joining unit and the infusion cannula is part of a cannula set.

9. A method according to any previous claim, further comprising determining an adjusted amount of the infusion fluid to be delivered after the disconnection has ended.

10. A method according to claim 9, wherein the second indication comprises information regarding the adjusted amount of infusion fluid.

11. A method according to claim 9 or 10 further comprising delivering the adjusted amount of infusion fluid after the disconnection has ended.

**12.** A method according to any previous claim, wherein the infusion fluid comprises insulin.

**13.** A method according to claim 1, wherein the amount of insulin not delivered because of the disconnection comprises of a sum of at least one intended dose of at least the following types: bolus, basal and extended bolus.

**14.** An apparatus comprising means for performing a method according to any of claims 1 to 13.

**15.** Computer-readable instructions which, when executed by computing apparatus, cause the computing apparatus to perform a method according to any of claims 1 to 13.

Figure 1

200    225   235     230     250

| RX | | TX | | |
| PRC. | | BATT. | | UI |
| MEM | | S/W | | |
| PUMP DEVICE | | INFUSION FLUID | | |

255
240
245

215
205    210
220

Figure 2

Figure 3

Figure 4

Figure 5

| Infusion pump 200 | Joining Unit 400 | Cannula set 300 |

| Timer Unit 600 | "Connection lost for 10 minutes" |

User interface 255

Infusion pump system 100

Figure 6a

Joining unit 400

Cannula set
300

Infusion Pump System
100

"The connection was lost
for 2 hours. Next 3 doses
of the medical substance
will be adjusted"

User interface 255

Figure 6b

Figure 6c

Figure 6d

Figure 6e

Current time: 16:15
CONNECTED

Time of disconnection: 13:00
Time of reconnection: 16:00
Duration of disconnection: 3h 00min
Insulin not delivered: 5 IU

255

Current time: 16:00
DISCONNECTED

Time of disconnection: 13:00
Time of reconnection: --:--
Duration of disconnection: 3h 00min
Insulin not delivered: 5 IU

255

Figure 6f

Receiving a first indication indicating a disconnection between a connector and an infusion cannula ——————— S1

Determining a duration of the disconnection ——————— S2

Determining, based on the determined duration of the disconnection, an amount of infusion fluid not delivered because of the disconnection between the connector and the infusion cannula ——————— S3

Providing a second indication indicating the detected disconnection ——————— S4

Figure 7

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/007970 A1 (MEDINGO LTD [IL]; YODFAT OFER [IL]; SHAPIRA GALI [IL]) 15 January 2009 (2009-01-15) * Par. 0012, 0033, 0035, 0041, 0050, 0057, 0069-0073, 0099; figure 2b * | 1-6,8-15 | INV. A61M5/168 G06F19/00 A61M39/10 A61M5/142 |
| X | US 2012/232521 A1 (BLOMQUIST MICHAEL L [US]) 13 September 2012 (2012-09-13) * Par. 0042, 0049, 0205, 0231-0237; figure 1 * | 1-3,7, 9-15 | |
| A | US 2012/029333 A1 (DOGWILER JOERG [CH] ET AL) 2 February 2012 (2012-02-02) * Par. 0071, 0081, 0082; figure 3 * | 1-15 | |

TECHNICAL FIELDS
SEARCHED (IPC)

A61M
G06F

The present search report has been drawn up for all claims

| Place of search | Date of completion of the search | Examiner |
|---|---|---|
| Munich | 17 January 2017 | Filippi, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 18 6204

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2017

| Patent document cited in search report | | Publication date | Patent family member(s) | | Publication date |
|---|---|---|---|---|---|
| WO 2009007970 | A1 | 15-01-2009 | EP | 2180909 A1 | 05-05-2010 |
| | | | JP | 2010533027 A | 21-10-2010 |
| | | | US | 2009048152 A1 | 19-02-2009 |
| | | | WO | 2009007970 A1 | 15-01-2009 |
| US 2012232521 | A1 | 13-09-2012 | CA | 2702843 A1 | 24-04-2008 |
| | | | EP | 2080131 A1 | 22-07-2009 |
| | | | US | 2008172026 A1 | 17-07-2008 |
| | | | US | 2012191062 A1 | 26-07-2012 |
| | | | US | 2012232484 A1 | 13-09-2012 |
| | | | US | 2012232485 A1 | 13-09-2012 |
| | | | US | 2012232486 A1 | 13-09-2012 |
| | | | US | 2012232521 A1 | 13-09-2012 |
| | | | US | 2012239362 A1 | 20-09-2012 |
| | | | US | 2012296269 A1 | 22-11-2012 |
| | | | US | 2014351712 A1 | 27-11-2014 |
| | | | WO | 2008048586 A1 | 24-04-2008 |
| US 2012029333 | A1 | 02-02-2012 | EP | 2353628 A2 | 10-08-2011 |
| | | | US | 2012029333 A1 | 02-02-2012 |

EPO FORM P0459

For more details about this annex : see Official Journal of the European Patent Office, No. 12/82